# EUROPEAN PATENT APPLICATION

(11) **EP 3 848 114 A1**
(43) Date of publication of application: **14.07.2021**
(21) Application number: 21152538.1
(22) Date of filing: 31.03.2015
(51) Int. Cl.: B01J 8/06, C07C 1/10, C07C 1/04, C07C 29/152, C07C 45/38

(54) **PROCESS AND PSEUDO-ISOTHERMAL REACTOR FOR PRODUCTION OF METHANOL**

(30) Priority: 02.04.2014 EP 14163204
(62) Divisional of application: 15714203.5
(71) Applicant: Haldor Topsøe A/S, 2800 Kgs. Lyngby (DK)
(72) Inventor: WIX, Christian, 2850 Nærum (DK); BOE, Michael, 2930 Klampenborg (DK); HANSEN, Anders Helbo, 3060 Espergærde (DK)
(74) Representative: Haldor Topsøe A/S

(57) **Abstract**

The present disclosure relates to a process and a process plant for production of methanol in a pseudo-isothermal flow reactor for comprising at least two reaction enclosures and a cooling medium enclosure configured to hold a cooling medium under pressure at a temperature proximate to the boiling point of said cooling medium, said reaction enclosures having an outer surface configured to be in thermal contact with the cooling medium, and each of said reaction enclosures having an inlet and an outlet with the associated benefit of enabling a two-stage pseudo-isothermal operation while only requiring a single cooling medium enclosure and only single cooling medium circuit.

## Description

The invention relates to a process for production of methanol employing pseudo-isothermal reactor having multiple reaction enclosures.

In exothermal processes a pseudo-isothermal reactor is often a relevant option since such a reactor can ensure optimum reaction conditions by providing a substantially constant cooling medium temperature. This is favorable where the process is limited by an exothermal equilibrium reaction in which equilibrium favors products at low temperature. The typical design of a pseudo-isothermal reactor involves a multitude of tubes inside a reactor shell filled with pressurized water, but other cooling media than water may also be used provided the boiling point is appropriate. The pressure on the cooling side controls the boiling point of the cooling medium, which then when operating at the boiling point may act as a heat sink at substantially constant temperature to the extent that liquid water is present in the reactor.

Pseudo-isothermal reactors are relatively complex and expensive pieces of equipment due to the need for materials and work related to providing multiple inlets and outlets and relatively large exterior surfaces as well as due to the operation of the cooling medium at elevated pressure. In addition the operation of multiple boiling water reactors requires the steam circuits of the individual reactors to be coupled, which adds complications and costs. Therefore the equipment costs may dictate that only a single pseudo-isothermal reactor is used, even though two isothermal stages would be a technically optimum choice.

It is known from the prior art to collect multiple reaction enclosures in a single heat exchange unit. GB 1 333 251 discloses a gas cooled reactor with two reaction enclosures for the production of methane. This reactor does not have the benefit of pseudo-isothermal operation which a boiling liquid cooled reactor provides, and accordingly the reactor does not consider the complications of coupling two steam circuits.

Common chemical processes benefitting from using pseudo-isothermal reactors include methane, methanol and formaldehyde production from synthesis gas, i.e. gas comprising hydrogen and carbon oxides and possibly other constituents. The synthesis gas may originate from a variety of sources, including from gasification of carbonaceous materials, such as coal, (typically heavy) hydrocarbons, solid waste and biomass; from reforming of hydrocarbons; from coke oven waste gas; from biogas or from combination of streams rich in carbon oxides and hydrogen - e.g. of electrolytic origin. Methane and methanol production are limited by an equilibrium involving a condensable component, and for formaldehyde production it is desirable to maintain the methanol concentration low due to inter alia considerations of explosion limits and catalyst stability. It may therefore be technically attractive to use two or more pseudo-isothermal reactors, in which a condensable product is often withdrawn between the reactors. In this way the reaction may take place at an optimum temperature while providing a more favorable equilibrium in the second stage. However, often such a process configuration involves excessive capital costs, and therefore it is not commonly used.

However, according to the present disclosure it is proposed to configure one pseudo-isothermal reactor shell with two or more reaction stages, which reduces the capital costs significantly.

In the following, a fluid, such as e.g. a fluid reactant or a fluid product shall be construed as comprising both gases and liquids.

In the following, the term isothermal reactor shall be construed as covering a cooled reactor operating at a substantially constant cooling medium temperature considering the reactor length and time, but allowing some variations on the process side. The term is interchanged with a pseudo isothermal reactor, a boiling liquid cooled reactor or a boiling water reactor, even though the practical implementation in form of a boiling liquid reactor may not be fully and ideally isothermal with a constant temperature throughout the reactor at all times. However, the cooling with boiling liquid provides a significant reduction in the temperature variation as a function of time and position compared to adiabatic reactors or reactors with interbed steam cooling. A pseudo isothermal reactor will typically have the cooling medium connected to a vapor circuit by means of a riser and a vapor drum, and the pressure in the vapor circuit will indirectly define the cooling medium temperature, since the pressure will define the cooling medium boiling point, and the reactor operates with the cooling medium at the boiling point.

In the following, a section of the reactor is called a reaction enclosure. However this shall not necessarily be construed as implying that a reaction takes place, since a reaction enclosure may simply have the function of a heat exchanger.

In the following, tubes shall be construed as enclosures of any circumferential shape and only characterized by being longer than the cross sectional distance. Typically tubes are cylindrical, but they may also have non-circular cross sectional shapes and a varying cross sectional shape over the tube length.

In the following, reference is made to a boundary wall of a reactor. This shall be construed as any wall at the surface of the reactor; i.e. a side wall, a bottom wall, a top wall or any other boundary wall without limiting the shape of such a reactor.

In the following the term "angular cylinder sector" is used. This term shall be construed as a subsection of a cylinder, having a ground plane being a sector of a circle, limited by an angular arc, i.e. a geometrical shape corresponding to a pie slice.

In the following reference is made to specific reactions without further definition. These reactions are well known to the person skilled in the art, but for an overview a short definition is given below.

In the following the methanation reaction or methanation process shall be construed as a process in which a feed comprising hydrogen and at least one carbon oxide such as carbon monoxide or carbon dioxide reacts according to equations (1) to (3) forming a gas rich in methane:

CO + H₂O = CO₂ + H₂ (1)

CO + 3 H₂ = CH₄ + H₂O (2)

CO₂ + 4 H₂ = CH₄ + 2 H₂O (3)

In the following methanol synthesis shall be construed as a process in which a feed comprising hydrogen and at least one carbon oxide such as carbon monoxide or carbon dioxide reacts according to equation (4) and (5) (and possibly the shift reaction (1)) forming a gas rich in methanol:

CO + 2 H₂ = CH₃OH (4)

CO₂ + 3 H₂ = CH₃OH + H₂O (5)

In the following formaldehyde synthesis shall be construed as a process in which a feed comprising methanol and oxygen reacts according to equation (6) forming a gas rich in formaldehyde:

2 CH₃OH + O₂ = 2 CH₂O + 2 H₂O (6)

In the following the module of a stream of synthesis gas containing hydrogen, carbon dioxide and carbon monoxide shall be defined as M= (H₂-CO₂) / (CO+CO₂) . For production of methane the stoichiometric module is 3, corresponding to reaction (2) and for production of methanol the stoichiometric module is 2 corresponding to reaction (4). A sub-stoichiometric amount of hydrogen is present when M is below 3, respectively 2 for the production of methane and methanol.

The present disclosure relates to a process plant for production of methanol comprising a pseudo-isothermal flow reactor for an exothermal reaction comprising at least two reaction enclosures and a cooling medium enclosure configured to hold a cooling medium under pressure at a temperature proximate to the boiling point of said cooling medium, said reaction enclosures having an outer surface configured to be in thermal contact with the cooling medium, each of said reaction enclosures having a reaction enclosure inlet and a reaction enclosure outlet and said cooling medium enclosure having a cooling medium inlet and a cooling medium outlet and each of said inlets and outlets being individually connectable with the associated benefit of enabling a two-stage pseudo-isothermal operation while only requiring a single cooling medium enclosure and only single cooling medium circuit.

In a further embodiment the reactor further comprises a catalytically active material inside at least 50% or 80% of the volume of at least one reaction enclosure, with the associated benefit of providing an optional pseudo-isothermal preheating section and a pseudo-isothermal reaction section for a catalyzed reaction. Preferably the volume of reaction enclosure holding the catalytically active material is fully enclosed in cooling medium.

In a further embodiment the reactor further comprises an inlet manifold wherein at least one of said first reaction enclosure and said second reaction enclosure comprises a multitude of reaction tubes, such as at least 2, 50, 100 or 1000 reaction tubes, each tube having a tube inlet in fluid connection with said inlet manifold, which is configured to receive a fluid stream from said reaction enclosure inlet and to distribute said fluid stream between the tube inlets of said multitude of reaction tubes, with the associated benefit of providing a high surface area relative to the volume of said reaction enclosure without complicating the inlet flow.

In a further embodiment the reactor further comprises an outlet manifold wherein at least one of said first reaction enclosure and said second reaction enclosure comprises a multitude of reaction tubes, such as at least 2, 50, 100 or 1000 reaction tubes, each tube having a tube outlet in fluid connection with said manifold, which is configured to receive a fluid stream from each of said multitude of tube outlets, said manifold combining the multitude of fluid streams into a single fluid stream and directing the single fluid stream to said reaction enclosure outlet, with the associated benefit of providing a high surface area relative to the volume of said reaction enclosure without complicating the outlet flow. Preferably the inlet and/or outlet manifolds are fastened by removable fastening means, such as screws and or bolts, such that the manifolds may be removed for exchange of catalyst and for service purposes.

In a further embodiment the reactor comprises a reactor divider separating the cooling medium from a reaction space in fluid connection with a reaction enclosure inlet or a reaction enclosure outlet and at least two reaction tubes of said reaction enclosure, thus defining an inlet manifold or an outlet manifold of said reaction enclosures, with the associated benefit of providing a manifold without requiring additional equipment while allowing free positioning of additional manifolds.

In a further embodiment the manifold comprises at least two substantially cylindrical manifold chambers, each manifold chamber being in fluid connection with the reaction tubes of a single reaction enclosure, and said two manifold chambers not being in direct fluid connection with each other, wherein said two cylindrical manifold chambers are positioned substantially concentrically, with the associated benefit of a simple mechanical configuration ensuring a high pressure stability.

In a further embodiment the manifold comprises at least two manifold chambers, each manifold chamber having the shape of an angular cylinder sector, and each manifold chamber being in fluid connection with the reaction tubes of a single reaction enclosure while not being in direct fluid connection with each other, and in which said at least two manifold chambers together define a cylindrical shape with the associated benefit of a simple mechanical configuration in which the individual loading of catalyst in each reaction enclosure is simplified.

In a further embodiment the reactor further comprises a means of fluid connection, said means of fluid connection being connected to a boundary wall of said reactor shell and to the inlet manifold or the outlet manifold, said means of fluid connection being configured to allow thermal contraction and expansion by a design such as a U tube or an angular tube, with the associated benefit of being robust against thermal contraction and expansion.

In a further embodiment the reactor further comprises a vapor drum having a vapor drum inlet and a vapor drum outlet, said a vapor drum inlet being in fluid communication by a riser tube with said cooling medium outlet and said vapor drum outlet being in fluid communication with said cooling medium inlet by a downcomer tube and said vapor drum being positioned above said cooling medium enclosure, with the associated benefit of said vapor drum having the function of separating the heated cooling medium in vapor form and liquid form.

A further aspect of the present disclosure relates to a reactor section comprising a reactor according to the present disclosure, said reactor section having a first reaction enclosure and a second reaction enclosure, and said reactor section further comprising a cooler and a gas/liquid separator having an inlet, a gas outlet and a liquid outlet, said reactor section being configured such that
a) the cooler inlet is in fluid communication with the first reaction enclosure outlet,
b) the cooler outlet is in fluid communication with the separator inlet, and
c) the separator gas outlet is configured to be in fluid communication with the second reaction enclosure inlet,
with the associated benefit of providing a cost effective pseudo-isothermal reaction system for an equilibrium limited reaction with a condensable product since the second stage is operated at reduced concentrations of the condensable product.

A process for the production of synthetic natural gas may also be carried out in a multiple reaction enclosure pseudo-isothermal reactor, said process comprising the steps of
a) directing a synthesis gas to contact a first material catalytically active in methanation inside a first methanation reaction enclosure,
b) withdrawing a first gas rich in methane and water from said first methanation reaction enclosure,
c) cooling said first gas rich in methane and directing it to a gas/liquid separator,
d) withdrawing a condensate and a methane rich gas from said separator,
e) directing said methane rich gas to contact a second material catalytically active in methanation inside a second methanation reaction enclosure, and
f) withdrawing a synthetic natural gas from said second methanation reaction enclosure,
with the associated benefit of the methane concentration in the product of the first methanation reaction enclosure being close to a first equilibrium, and subsequently enabling a further increase in methane concentration by shifting the equilibrium limitation through removal of the product water.

A process for production of synthetic natural gas may alternatively be carried out where said multiple reaction enclosure pseudo-isothermal reactor further comprises a shift reaction enclosure containing a material catalytically active in the water gas shift reaction, said process further comprises the step prior to step a) of directing a synthesis gas rich in CO and H₂O to said shift reaction enclosure providing a synthesis gas to be fed to said first methanation reaction enclosure, with the associated benefit of reducing the adiabatic equilibrium temperature in the following reaction enclosure by carrying out part of the exothermal reaction in the first reaction enclosure.

The process for the production of synthetic natural gas may alternatively be carried out with an amount of said first gas rich in methane and water being combined with said synthesis gas as a feed to said first methanation reaction enclosure or combined with a CO rich synthesis gas feeding said synthesis gas rich in CO and H₂O to said shift reaction enclosure, with the associated benefit of providing water for the reaction; either in form of a reactant for the shift reaction or for limiting the exothermal reaction by the use of water with the additional effect of reducing the amount of process condensate.

A further aspect of the present disclosure relates to a process for production of methanol in a multiple reaction enclosure pseudo-isothermal reactor, said process comprising the steps of
a) directing a synthesis gas to contact a first material catalytically active in methanol synthesis inside a first reaction enclosure,
b) withdrawing a first gas rich in methanol from said first reaction enclosure,
c) cooling said first gas rich in methanol and directing it to a gas/liquid separator,
d) withdrawing a condensate and an intermediate process gas from said separator,
e) directing said intermediate process gas to contact a second material catalytically active in methanol synthesis inside a second reaction enclosure, and
f) withdrawing a second product gas rich in methanol from said second reaction enclosure,
with the associated benefit of the methanol concentration in the product of the first reaction enclosure being close to a first equilibrium, and subsequently enabling a further methanol production by shifting the equilibrium limitation through removal of the methanol product from said first gas rich in methanol.

In yet another embodiment of the process for production of methanol, said process further comprises a sulfur guard reaction enclosure comprising a material active in desulfurization for withdrawing sulfur from said synthesis gas upstream said other reaction enclosures, with the associated benefit of said sulfur guard reaction enclosure being preheated by the boiling cooling medium and thus being ready for operation.

A process for production of formaldehyde may also be carried out in a multiple reaction enclosure pseudo-isothermal reactor, said process comprising the steps of
a) directing a feed gas comprising methanol and oxygen to contact a first material catalytically active in formaldehyde synthesis inside said first reaction enclosure,
b) withdrawing a first product gas rich in formaldehyde from said first reaction enclosure,
c) combining said product rich in formaldehyde with a further feed comprising methanol forming an intermediate process gas,
d) directing said intermediate process gas to contact a second material catalytically active in formaldehyde synthesis inside said second reaction enclosure, and
e) withdrawing a second product gas rich in formaldehyde from said second reaction enclosure, with the associated benefit of cooling said first product gas without the use of heat exchange equipment, and the additional benefit of maintaining a low concentration of methanol, which would otherwise have required a process including a cooling step and recycling of a product gas with an associated increase in energy consumption and an increased process volume.

For exothermal equilibrium limited processes pseudo-isothermal reactors are often favorable to use. However, a pseudo-isothermal reactor is typically an expensive piece of equipment, and therefore the use of such reactors is often limited to the cases only where the benefit is extensive.

According to the present disclosure a new configuration of pseudo-isothermal reactors is provided, according to which two or more sections (i.e. reaction enclosures) of the reactor may be fed independently, thus simplifying the design of multiple pseudo-isothermal stages significantly by providing a design in which multiple reaction enclosures can be operated with a single cooling medium enclosure.

A common concept for a pseudo-isothermal reactor is the so-called boiling-liquid-cooled reactor (often called a boiling water reactor), in which the cooling medium is a liquid, typically water, but it may also be e.g. oil or salt, said liquid being in thermal contact with one or more reaction enclosures, such as tubes. The liquid is pressurized, and the pressure controls the boiling point of the cooling medium, which thus is kept at a substantial constant temperature close to the boiling point of the liquid, where excess energy is removed as vaporization enthalpy e.g. by evaporation of liquid water into steam. In this way the process side of the reaction enclosures is in thermal contact with a cooling medium having substantially the same temperature along the entire length of the reactor and substantially the same temperature over time (as long as the pressure is not modified). Such a boiling-liquid-cooled reactor significantly reduces the variation in temperature on the process side, even though hot spots may exist where very rapid exothermal reactions take place.

If two so-called boiling water reactors are to be operated at the same temperature, cost may be reduced through integration of the steam drum circuits, but this integration must be made carefully to avoid unintended pressure conflicts.

However by providing a pseudo-isothermal reactor with two or more reaction enclosures sharing a cooling medium enclosure it is possible to obtain multiple savings, especially in connection with small to medium sized pseudo-isothermal reactors. First of all only a single pressurized cooling medium enclosure is required, and furthermore a single and simple steam circuit is rendered possible.

Often the reactions taking place in a pseudo-isothermal reactor are catalyzed by a heterogeneous catalyst, but the reactions may also take place in the gas phase. The reaction enclosure may or may not contain catalytically active material in the majority of its volume or only contain catalytically active material in a partial volume. If only a partial volume of the reaction enclosure contains catalytically active material this volume may be considered a heat exchanger section for pre-heating the reacting fluid prior to contacting the catalytically active material. If such a pre-heating section is included, it may be configured to have a higher flow rate at the tube wall to increase the heat transfer, e.g. by providing an appropriate tube insert.

The preheating of reactants during the reactor start-up is a further benefit associated with a pseudo-isothermal reactor. During the start-up the cooling medium may in such a reactor have the function of a heating medium and be heated to an appropriate temperature externally, with the associated benefit of providing activation energy for the reaction.

A multi-stage pseudo-isothermal reactor may comprise two, three or more reaction enclosures, each reaction enclosure comprising one or a multitude of reaction tubes configured for substantially parallel operation. The number of reaction tubes in each reaction enclosure may be identical or it may differ between the reaction enclosures, and similarly the tubes may be identical or vary in design, such as diameter and shape. The external shape of some or all of the reaction tubes may be designed with an efficient heat exchange in mind, e.g. by having cooling fins or by having a shape contributing to increased turbulence. For the tubes to operate successfully in parallel, one or more inlet manifolds and/or outlet manifolds may be required for simplicity. Such a manifold must be designed with proper consideration of the flow characteristics, including the mixing and pressure drop. Furthermore the fluid connection from the inlet to the manifold or the individual tubes may be configured to allow a thermal expansion and contraction by having appropriate bends to relieve the interface to the boundary wall of the reactor.

The reaction enclosures may be defined by a number individual tubes connected to a manifold, but for practical reasons, such as compensating for pressure loss or providing heat exchange a reaction enclosure may also comprise a section with higher or lower residence time. One way of implementing this is a drum shaped reaction enclosure inside which a number of tubes holding cooling medium is positioned. This may be configured such that the height of the drum shaped reaction enclosure is lower than the other reaction enclosure, with a reduced pressure loss as a resulting benefit.

The design of manifolds for a multistage reactor requires several considerations. The manifold must be designed appropriately considering pressure differences between the reaction tubes and the reaction enclosures. Furthermore in the event a reaction tube contains catalytically active material it must be considered how to load this material to the reaction tube, and the life cycle of the catalyst must also be taken into consideration.

With respect to catalytically active material three general types of catalytically active material can be considered; catalytically active pellets, catalytically active monoliths and catalyzed hardware. The nature of the catalytically active material may be the same or different between the individual reaction enclosures and/or the individual reaction tubes.

Catalytically active pellets (which may be produced by many processes, including extrudation or pelleting) are the most common type of industrial catalysts, and they are often used in processes where there is a risk of catalyst deactivation, which may require occasional or regular replacement of the catalysts.

Catalyzed hardware is a concept described inter alia in EP0855366, according to which the surface of the structural materials such as tubes is modified, e.g. by application of a catalytic film making the surface catalytically active. Reaction tubes provided as catalyzed hardware are not expected to be replaced since the use of catalyzed hardware is typically related to applications with little tendency to deactivation of the catalysts.

Structured catalysts such as catalytically active monoliths are also complex to replace and are therefore primarily used in processes where the catalyst is not deactivated significantly.

As opposed to catalyzed hardware and catalytically active monoliths, catalytically active pellets are often used in processes which require replacement of the catalyst. The manifold for pseudo-isothermal reactors used in reaction systems involving catalytically active pellets must be structured in a manner allowing access to the catalyst pellets.

When the manifold is structured to allow replacement of catalytically active material, the replacement may be carried out individually in the reaction tubes or more conveniently in the group of reaction tubes comprised in the same reaction enclosures.

A structure may define where each group of reaction tubes comprised in the same reaction enclosure is positioned concentrically. Such a structure may be beneficial from a pressure perspective and a production consideration since it enables a manifold design, where concentric cylindrical manifold chambers are used for defining the inlet and/or the outlet of the reaction enclosures in such a manner that each reaction enclosure only interfaces neighboring enclosures. This is beneficial especially with respect to a practical production consideration.

However, from the perspective of being able to access the tubes in a reaction enclosure for loading and/or unloading catalyst material, it may be more beneficial to structure the manifold chambers as pie-shaped, angular cylinder sectors of an appropriate size, which then may be tightened against a reactor top plate or bottom plate.

A further alternative is beneficial from the perspective of loading and unloading catalyst material, said alternative being the definition of "island" manifolds, where each island manifold covers a defined number of neighboring reaction tubes.

All types of manifolds may be combined with a single manifold, in which the inlet or outlet of a number of tubes is in fluid connection with a volume defined by a tube sheet and the boundary wall of the reactor.

At the bottom of the reaction enclosures or the reaction tubes a mesh, a perforated sheet or another particle-withholding element, such as balls made from ceramics or another inert material, may be provided for fixation of said catalytically active material, especially when this material is in form of catalytically active pellets.

When using a pseudo-isothermal reactor compared to an adiabatic reactor, the desired effects are mainly related to three aspects. One aspect is the possibility of influencing the equilibrium in exothermal reactions where an elevated temperature favors reactants. A second aspect is the possibility of ensuring that the catalyst and/or products are kept below critical temperatures such that thermal damage of the catalyst is avoided. A third aspect has the effect that reactions forming side products or consuming products at elevated temperatures are often reduced at low temperatures.

An important problem applies specifically to materials being catalytically active in methanation carbon formation, and this problem may be solved or reduced by limiting the maximum temperature through the use of a pseudo-isothermal reactor. In WO2012/084076 a correlation is established between the ratio of steam to higher hydrocarbons ratio and the temperature, said correlation identifying a region in which methanation can take place safely as well as a region above the so-called carbon limit in which there is a risk of carbon formation.

The risk of whisker carbon formation in a pseudo-isothermal methanation reactor is reduced compared to an adiabatic reactor operating with the same feed gas composition and inlet temperature as the peak temperature is lower than in the isothermal reactor as heat is removed from the reactor.

The risk of carbon formation from higher hydrocarbons is reduced for the same reasons as stated above, but also because of a relatively high water concentration at the temperature peak as a consequence of the heat removal.

For similar reasons a pseudo-isothermal reactor may also be beneficial in relation to reactions in which the catalytically active material is deactivated thermally e.g. by sintering, since the excessive temperature is limited to only an initial section of the reaction tubes before the process side is cooled by the cooling medium.

In some process configurations employing multiple stage reactions the module of the feed to the two stages may beneficially be different. Typically the amount of hydrogen in the feed to the first stage is deliberately superstoichiometric (i.e. the module M is above 2 for methanol production and above 3 for methane production) and the amount of hydrogen in the later stage is substoichiometric (i.e. the module M is below 2 for methanol production and below 3 for methane production). The associated benefit is that the formation of by-products such as ethanol, ketones and aldehydes (in the case of methanol production) and carbon deposits (in the case of methane production) is reduced by ensuring excess availability of hydrogen in the reactive first stage.

These features and benefits of the present disclosure are described in greater details below with reference to the accompanying drawings, which are intended for illustration purposes and not limiting the scope of the application. Features and elements shown in the Figures may not be essential to the present disclosure, or not even to the individual embodiment of the disclosure, and similarly, additional features of secondary relevance to the disclosure, such as features related to the efficient heat integration of processes, may be omitted in embodiments corresponding to the present disclosure, or may have been omitted in the illustrated embodiments. While similar items have been numbered similarly in the Figures for the ease of understanding, a similarity in numbers shall not be construed as a similarity of features unless described as such.

### Figures

Fig. 1 illustrates an embodiment of a pseudo-isothermal reactor according to the present disclosure.
Fig. 2 illustrates specific details of the reactor and manifold.
Fig. 3 illustrates a methanation process with a sulfur guard and four adiabatic methanation reactors.
Fig. 4 illustrates a methanation process with a sulfur guard, one pseudo-isothermal and one adiabatic methanation reactor.
Fig. 5 illustrates a methanation process with a sulfur guard and two pseudo-isothermal methanation reactors.
Fig. 6 illustrates a methanation process with a sulfur guard and a dual stage pseudo-isothermal methanation reactor.
Fig. 7 illustrates a methanation process with a triple stage pseudo-isothermal reactor.
Fig. 8 illustrates a coke oven gas methanation process with three adiabatic reactor.
Fig. 9 illustrates a coke oven gas methanation process with a dual stage pseudo-isothermal reactor.
Fig. 10 illustrates a methanol synthesis process with a sulfur guard and a single stage pseudo-isothermal reactor .
Fig. 11 illustrates a methanol synthesis process with a sulfur guard and a triple stage pseudo-isothermal reactor according to the present disclosure.
Fig. 12 illustrates a methanol synthesis process with a sulfur guard and a dual stage pseudo-isothermal reactor and recycle according to the present disclosure.
Fig. 13 illustrates a formaldehyde synthesis process with a dual stage pseudo-isothermal reactor.
Fig. 14 illustrates a specific mechanical configuration of a dual stage pseudo-isothermal reactor according to the present disclosure.

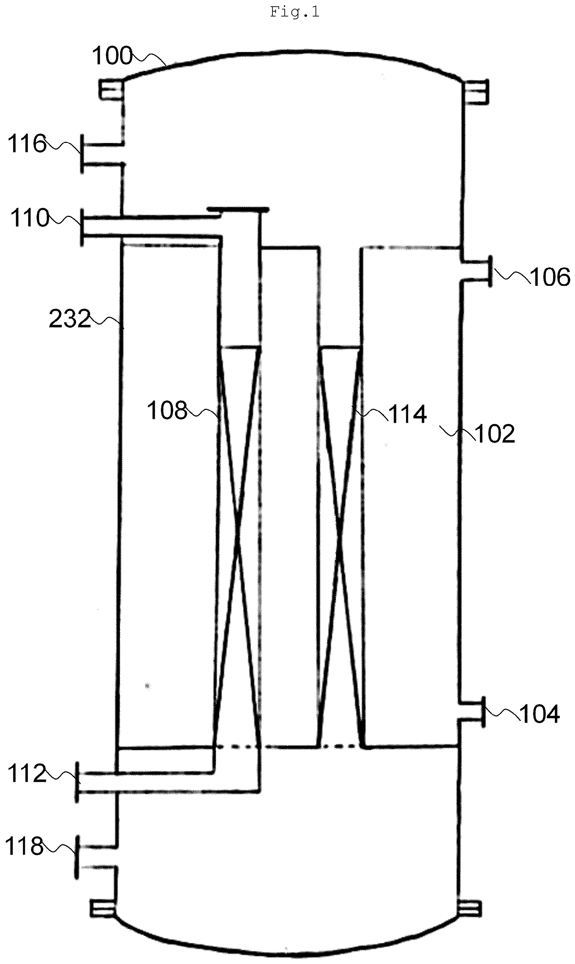

In Fig. 1 a pseudo-isothermal reactor 100 is shown. For simplicity reasons only a single reaction tube 114 is shown in the primary reaction enclosure, and only a single reaction tube 108 is shown in the secondary reaction enclosure. The reactor comprises a cooling medium enclosure 102 with a cooling medium inlet 104 and a cooling medium outlet 106, which typically are designed for keeping the cooling medium under elevated pressure. The cooling medium enclosure 102 houses a first reaction enclosure consisting of a single reaction tube 114 and a second reaction enclosure consisting of a single reaction tube 108. Each reaction enclosure comprises an inlet 110, 116 and an outlet 112,118. The embodiment illustrated here includes both a section of the first reaction enclosure for pre-heating the reacting fluid and a section for holding a catalytically active material, but a reaction enclosure may contain only a pre-heating section or a section for holding a catalytically active material.

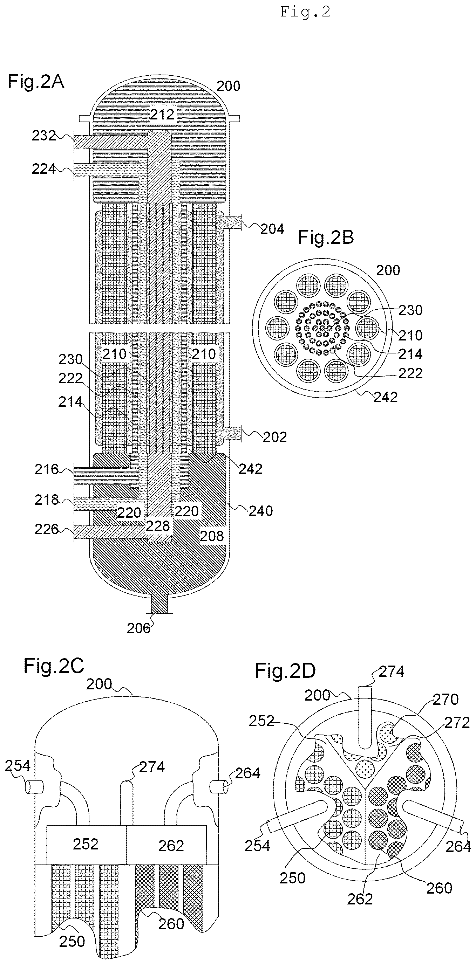

Fig. 2A shows an example of a pseudo-isothermal reactor with concentrically organized reaction tubes according to the present disclosure. The reactor comprises a cooling liquid enclosure having a cooling liquid inlet 202 and a cooling medium outlet 204. A first reaction enclosure inlet 206 leads to a first reaction enclosure manifold 208 defined by the boundary wall 240 and a tube sheet 242 from which the process gas is directed to the tubes of the first reaction enclosure 210 which here are illustrated as comprising monolithic catalyst. The outlet of the first reaction enclosure is connected to a second reaction enclosure manifold 212 defined similarly and in fluid connection with the tubes of the second reaction enclosure 214 and in connection with a second reaction enclosure outlet 216. An inlet 218 is connected to a third reaction enclosure manifold 220 in turn connected to the tubes of the third reaction enclosure 222 and further connected to a third reaction enclosure manifold and outlet 224. An inlet 226 is connected to a fourth reaction enclosure manifold 228 connected to the tubes of the fourth reaction enclosure 230 and further connected to a third reaction enclosure manifold and outlet 232.

Fig. 2B shows a top view of the central part of the same reactor 200 with the tubes of the first reaction enclosure 210, the tubes of the second reaction enclosure 214, the tubes of the third reaction enclosure 222 and the tubes of the fourth reaction enclosure 230.

An alternative configuration of the manifolds of a similar reactor 200 is shown in Fig. 2C (side view) and Fig. 2D (top view) in which the reaction tubes 250, 260, 270 are organized in 3 angular cylinder sectors each connected to a reaction section outlet 254, 264, 274 through a manifold 252, 262, 272. This type of manifold may be better suited for filling the individual reaction enclosures with catalytically active pellets, especially if the sections contain different types of catalytically active pellets.

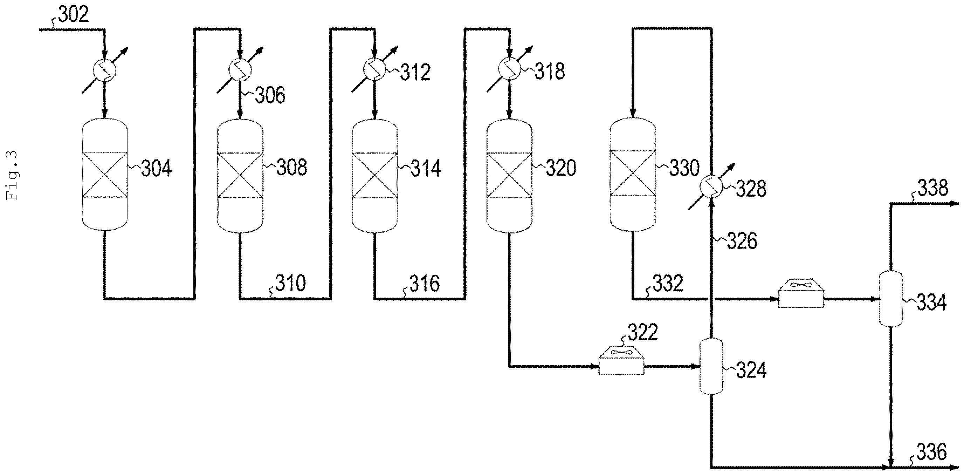

Fig. 3 illustrates a methanation process with four adiabatic methanation reactors 308, 314, 320, 330 according to the prior art. Synthesis gas 302 is directed to a sulfur guard 304, which is optional if the catalyst is insensitive to sulfur or sulfur is absent in the synthesis gas. The synthesis gas 302 is directed to a pre-heater providing a desulfurized synthesis gas 306 and further to a first methanation reactor 308 at an appropriate temperature so as to provide a first methane rich gas 310.

The first methane rich gas is cooled in a heat exchanger 312 and allowed to react further in a second methanation reactor 314 and a third methanation reactor 320 involving an intermediate cooling 318 of the second stage methane rich gas 316. Prior to a fourth and final methanation reactor 330, water 336 is condensed after cooling 322 in a separator 324 to shift the reaction equilibrium of the final methanation feed gas 326 before the water in the final stage methane rich gas 332 is separated in 334 and a synthetic natural gas 338 is produced.

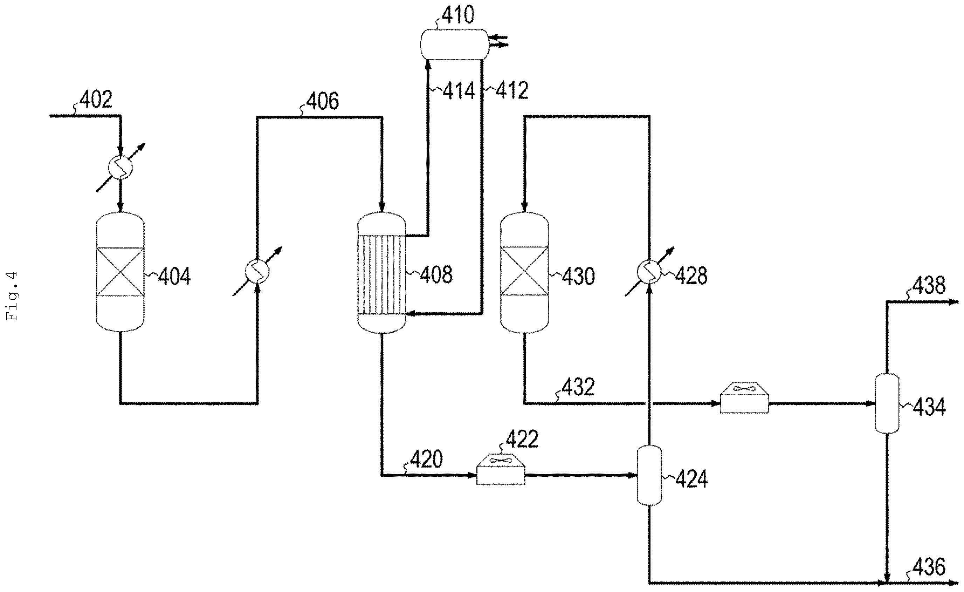

Fig. 4 illustrates a methanation process with one pseudo-isothermal reactor 408 and one adiabatic reactor 430 according to the prior art. The process comprises preheating a synthesis gas 402 and feeding it to an optional sulfur guard 404. Subsequently the desulfurized synthesis gas 406 is directed to the pseudo-isothermal reactor 408 producing a first methane rich gas 420, which is cooled 422 followed by a separation 424 of water. As a result a dry methane rich gas is obtained which is heated 428 and directed to the final methanation stage 430 in a manner similar to the one illustrated in Fig. 3.

The final methane rich gas 432 is cooled prior to separation 434 into water 436 and synthetic natural gas 438. The cooling medium 412 of the pseudo-isothermal reactor 408 is water, which is outlet from the pseudo-isothermal reactor as a steam/water mixture 414 and directed to a steam drum 410, in which the pressure is controlled to indirectly control the temperature of the boiling water inside the pseudo-isothermal reactor.

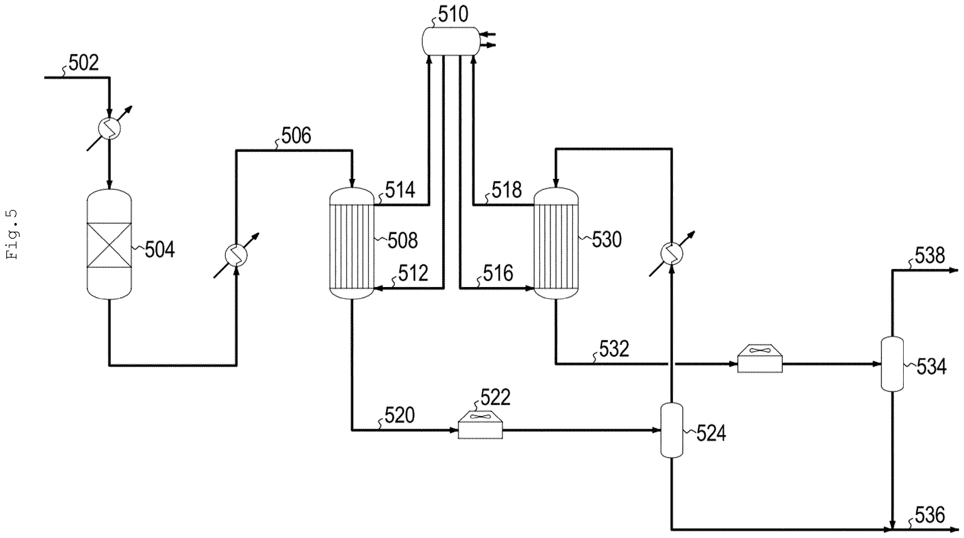

In Fig. 5 a methanation process with two pseudo-isothermal reactors 508, 530 is illustrated. The process comprises feeding preheated synthesis gas 502 to an optional sulfur guard 504, and directing the desulfurized synthesis gas 506 to a first pseudo-isothermal reactor 508 with cooling 522 and separation 524 of a first methane rich gas 520 prior to the final stage 530, which provides a final methane rich gas 532 which is cooled and separated 534 in water 536 and synthetic natural gas 538 as shown in Fig. 3 and Fig. 4. The cooling medium 512,516 of the pseudo-isothermal reactors 508, 530 is water, which exits the pseudo-isothermal reactor in form of steam 514, 518 directed to a steam drum 510. The pressure in the steam drum indirectly controls the temperature of the boiling water inside the cooling medium enclosures of the pseudo-isothermal reactors 508, 530. As only a single steam drum 510 is included, the operational temperature of the two reactors 508, 530 must be the same. Furthermore to balance the flow and pressure, the water lines 512, 516 and steam lines 514, 518 must be carefully designed to be symmetrical.

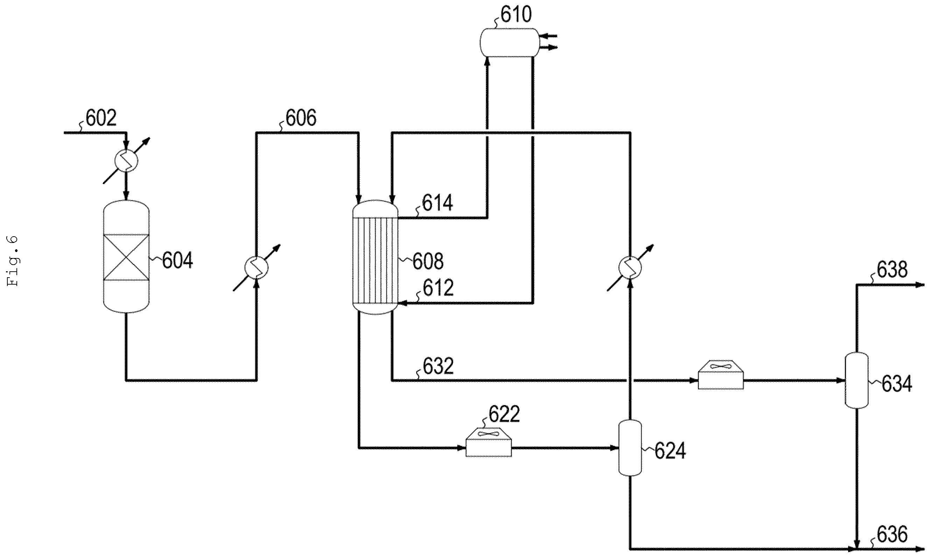

In Fig. 6 a methanation process with a dual stage pseudo-isothermal reactor 608 is illustrated. The process comprises feeding preheated synthesis gas 602 to an optional sulfur guard 604, and directing the desulfurized synthesis gas 606 to a first pseudo-isothermal reaction enclosure 608, and to cooling 622 and condensation 624 prior to the final reaction enclosure of the pseudo-isothermal reactor 608 which provides a final methane rich gas 632 which is cooled and separated 634 into water 636 and synthetic natural gas 638 as shown in Fig. 3, Fig. 4 and Fig. 5. The cooling medium 612 of the pseudo-isothermal reactor 608 is water, which exits the pseudo-isothermal reactor 608 as steam 614 directed to a steam drum 610, in which the pressure is controlled, to indirectly control the temperature of the boiling water inside the pseudo-isothermal reactor. As the cooling medium enclosure is common to the two reactor stages, a balancing of the steam lines is not required. From a process perspective the process of Fig. 6 corresponds fully to the process of Fig. 5, but the required equipment has been significantly simplified.

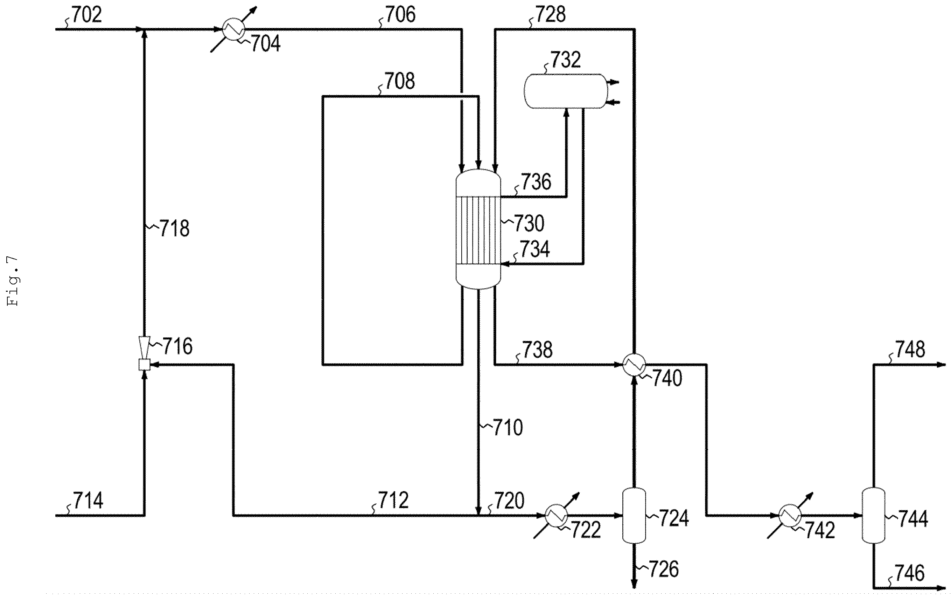

In Fig. 7 a methanation process with a triple stage pseudo-isothermal reactor 730 is illustrated. Synthesis gas comprising CO and CO₂ 702 is mixed with a mixture of steam and methane rich gas 718 in order to obtain the first reactor feed gas 706. To avoid the cost of a recycle compressor, the recycle is driven by an ejector 716 with steam 714 as motive gas. The first reactor feed gas 706 is first heated 704 and then directed to a first reaction enclosure containing a material catalytically active in the water gas shift reaction in a pseudo-isothermal reactor 730 providing a first methanation feed gas 708. This first methanation feed gas 708 is directed to a second reaction enclosure in a pseudo-isothermal reactor 730 providing a first methane rich gas 710. The recycled 712 part of the first methane rich gas is together with steam 714 directed to the ejector 716 providing the recycle stream 718. The part of the first methane rich gas not being recycled 720 is cooled 722 and directed to a separator 724,and a process condensate 726 is withdrawn to obtain a dried final methanation reactor feed gas 728 which is heated 740 and directed to a third reaction enclosure of the pseudo-isothermal methanation reactor 730. From the methanation reactor 730 the final methane rich gas 738 is withdrawn, cooled 740, 742 and separated 744 in the process condensate 746 and the substitute natural gas 748. In practice, the feed stream 702 must be substantially absent of sulfur or the catalytically active material must be of a type resistant to sulfur.

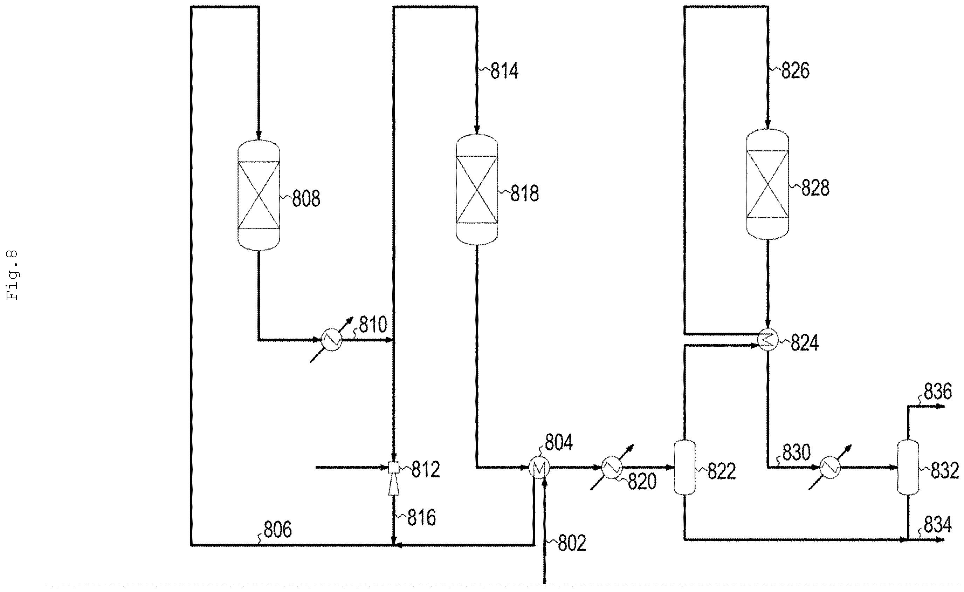

In Fig. 8 a three stage methanation process is illustrated. The process receives a coke oven gas in form of a feed stream 802, which is preheated 804. Since this gas comprises C2 and C3 hydrocarbons the risk of carbon formation is further increased, and therefore a combination of steam and recycled methane rich gas 816 is added to the feed gas. To avoid the cost of a recycle compressor, the recycle is driven by an ejector 812 with steam as motive gas. The methanation feed gas 806 is directed to a first methanation reactor 808, from which a first methane rich gas 810 is withdrawn and cooled. The not recycled part 814 of the first methane rich gas is directed to a second methanation reactor 818. A second methane rich gas is withdrawn from the second methane reactor 818 and cooled. In this manner heat is provided for preheating the feed stream in 804. The cooled second methane rich gas is further cooled 820 and directed to a separator 822, and a process condensate 834 is withdrawn to obtain a dried second methane rich gas which is preheated 824 to provide a final methanation reactor feed gas 826. The reactor feed gas 826 is directed to the final methanation reactor 828 from which the final methane rich gas 830 is withdrawn, cooled and separated 832 in the process condensate 834 and the substitute natural gas 836. In practice the feed stream 802 must be substantially absent of sulfur, or the catalytically active material must be of a type resistant to sulfur, or a desulfurization reactor must be included.

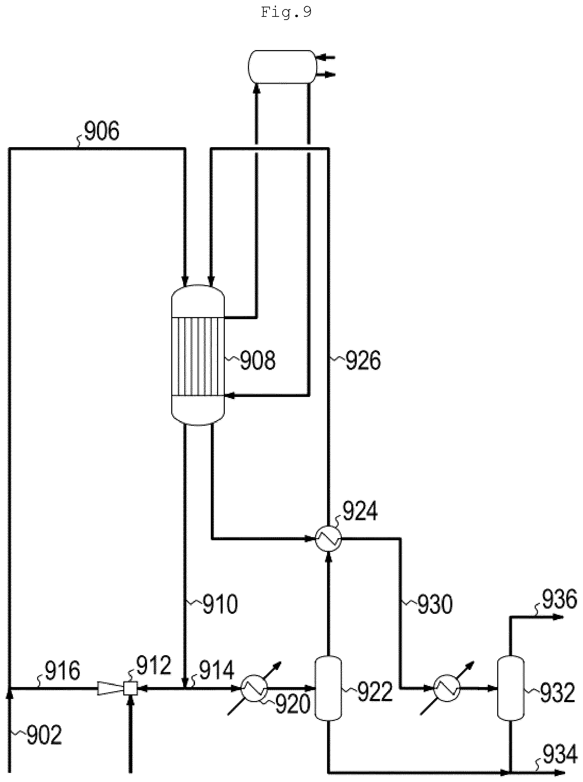

In Fig. 9 a methanation process is shown, said process being similar to the one shown in Fig. 8. Again the process is receiving a coke oven gas as feed stream 902. Since this gas comprises C2 and C3 hydrocarbons the risk of carbon formation is further increased, and therefore a combination of steam and recycled methane rich gas 916 is added to the feed gas. To avoid the cost of a recycle compressor the recycle is driven by an ejector 912 with steam as motive gas. The methanation feed gas 906 is directed to a first reaction enclosure in a pseudo-isothermal methanation reactor 908, from which a first methane rich gas 910 is withdrawn. The not recycled part 914 of the first methane rich gas is cooled 920 and directed to a separator 922, and a process condensate 934 is withdrawn to obtain a dried first methane rich gas which is preheated 924 to provide a final methanation reactor feed gas 926. The final methanation reactor feed gas is directed to the second reaction enclosure of the pseudo-isothermal methanation reactor 908 from which the final methane rich gas 930 is withdrawn, cooled by pre-heating the final methanation reactor feed gas in 924 and separated 932 in the process condensate 934 and the substitute natural gas 936. In practice, the feed stream 902 must be substantially absent of sulfur, or the catalytically active material must be of a type resistant to sulfur, or a desulfurization reactor must be included.

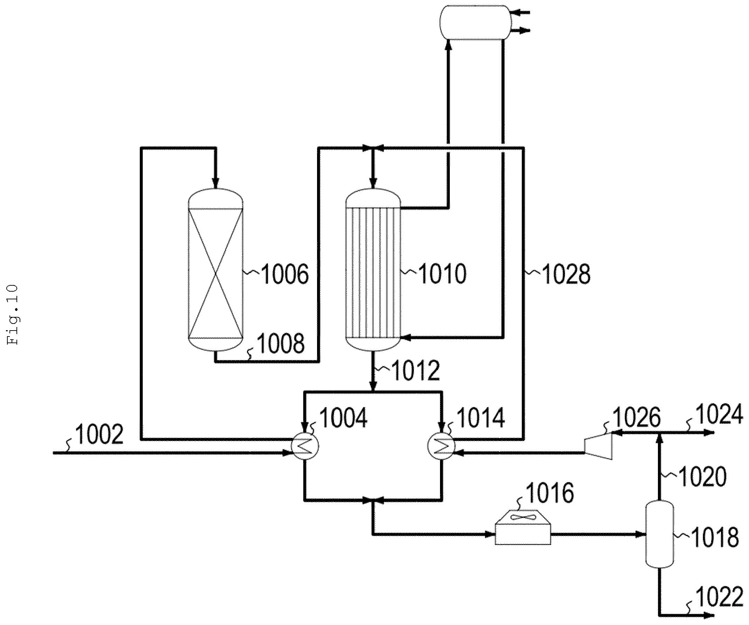

In Fig. 10 a methanol synthesis process with a single pseudo-isothermal reactor and recycle according to the prior art is illustrated. According to the process scheme, synthesis gas 1002 is preheated by a heat exchanger 1004, and directed to a sulfur guard 1006 which is optional if the catalyst is insensitive to sulfur or sulfur is absent in the synthesis gas. As a result a feed gas 1008 is provided which is combined with a recycle stream 1028 and directed to a pseudo-isothermal reactor 1010, in which the synthesis gas reacts to form a heated methanol rich gas 1012. This gas is cooled in heat exchangers 1004, 1014 and further in a cooler 1016 prior to condensation and separation in a gas/liquid separator 1018 into a methanol product 1022 and an unconverted synthesis gas 1020. A portion of the unconverted synthesis gas 1020 is directed to be purged 1024, and another portion is compressed 1026 and recycled 1028.

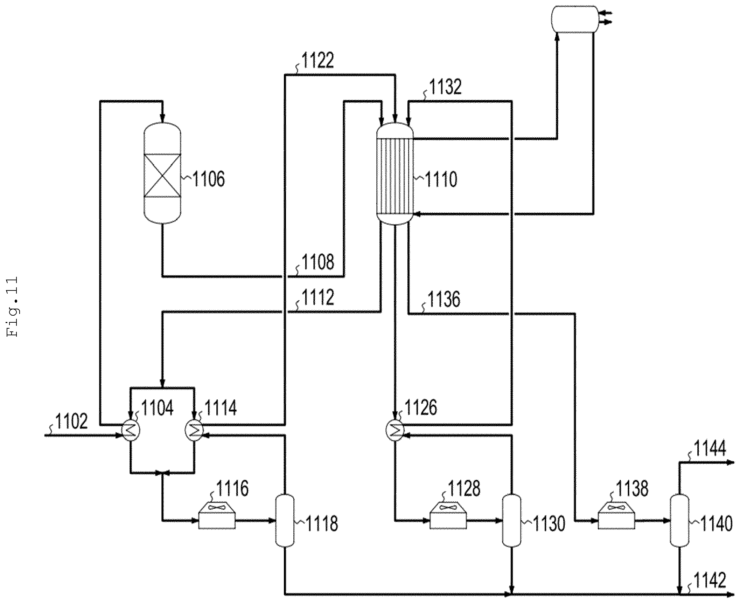

In Fig. 11 a methanol synthesis process with a triple stage pseudo-isothermal reactor according to the present disclosure is illustrated. According to the process scheme, synthesis gas 1102 is preheated in a heat exchanger 1104 and directed to a sulfur guard 1106, which is optional if the catalyst is insensitive to sulfur or sulfur is absent in the synthesis gas 1102. As a result a desulfurized synthesis gas 1108 is provided and directed further to a first pseudo-isothermal reaction enclosure of a triple stage pseudo-isothermal reactor 1110, in which the desulfurized synthesis gas 1108 reacts to form a first hot methanol rich gas 1112.

This first hot methanol rich gas 1112 is cooled in a first pair of heat exchangers 1104, 1114 and further by a cooler 1116, prior to condensation of methanol 1142 for shifting the equilibrium of the reacting gas 1122. The reacting gas 1122 is preheated 1114 and directed to a second pseudo-isothermal reaction enclosure of the triple stage pseudo-isothermal reactor 1110, with a similar cooling 1126, 1128, condensation and separation in a gas/liquid separator 1030 and reheating 1126 setup before being directed as a final feed gas 1132 to a third pseudo-isothermal reaction enclosure providing a final methanol rich gas 1136, followed by cooling 1138 condensation and separation in a gas/liquid separator (1140 of methanol 1142 and the unconverted synthesis gas 1144. By operation of the three reaction enclosures under pseudo-isothermal conditions it becomes possible to employ the synthesis gas far more effectively.

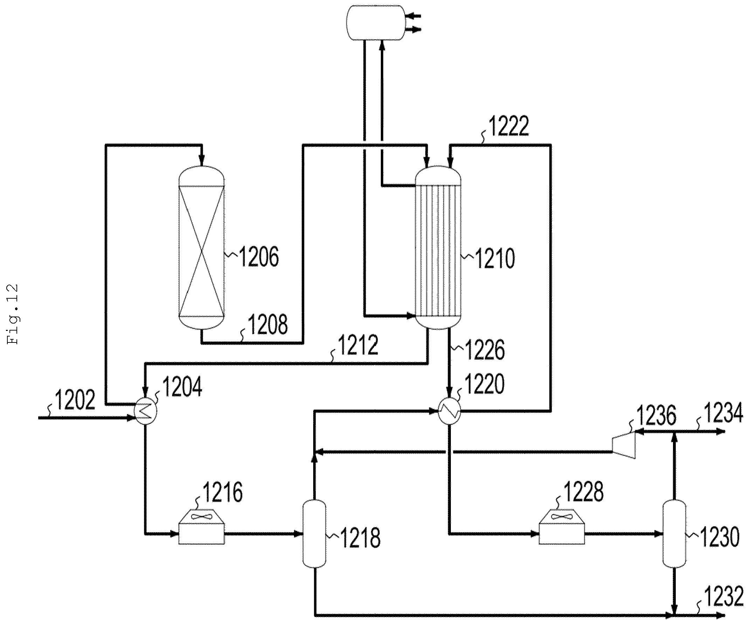

In Fig. 12 a methanol synthesis process with a two stage pseudo-isothermal reactor and recycle according to the present disclosure is illustrated. According to the process scheme, synthesis gas 1202 is preheated by a heat exchanger 1204 and directed to a sulfur guard 1206 which is optional if the catalyst is insensitive to sulfur or sulfur is absent in the synthesis gas 1202. The optionally desulfurized synthesis gas 1208 is directed further to a first pseudo-isothermal reaction enclosure of a dual stage pseudo-isothermal reactor 1210, in which the optionally desulfurized synthesis gas 1208 reacts to form a first hot methanol rich gas 1212.

This first hot methanol rich gas 1212 is cooled in a first heat exchanger 1204 and further by a cooler 1216, prior to separation 1218 of methanol 1232 for shifting the equilibrium of the reacting gas 1222. The reacting gas 1222 is preheated 1220 and directed to the second pseudo-isothermal reaction enclosure of the dual stage pseudo-isothermal reactor 1210 providing a final methanol rich gas 1226 to a similar cooling 1220, 1228, condensation and separation in a gas/liquid separator 1230 setup. Subsequently, the gas phase is split and directed to purge 1234 and a recycle compressor 1236 and the liquid phase is withdrawn in form of methanol product 1232.

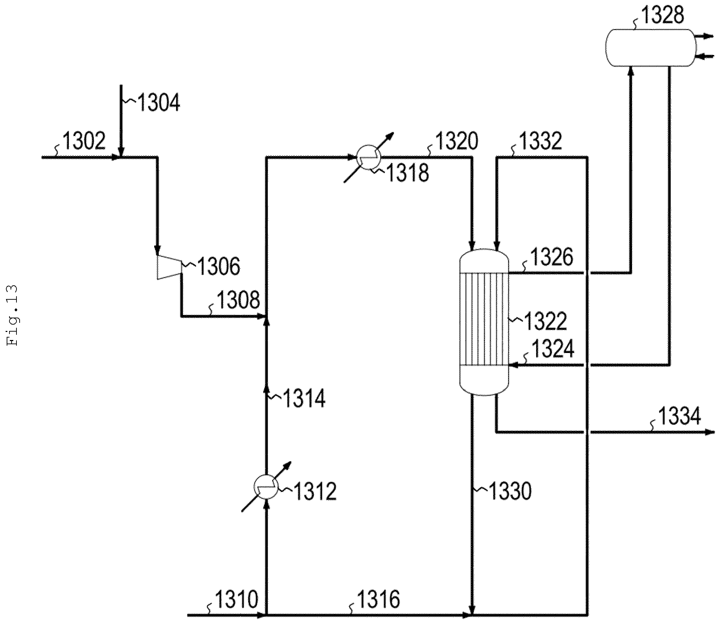

In Fig. 13 a formaldehyde synthesis process with a two stage pseudo-isothermal reactor is illustrated. According to the process scheme air 1302 is mixed with recycle gas 1304 and directed to a compressor 1306 providing the first synthesis gas mixture 1308. Methanol 1310 is added to the process in two stages. The part of methanol added to the process first 1314 is heated 1312 before mixing with the first synthesis gas mixture 1308 to obtain the first formaldehyde reactor feed gas 1320. The reactor feed gas 1320 is heated 1318 and directed to the formaldehyde reactor 1322, from which a first formaldehyde rich gas 1330 is withdrawn. The second part of the methanol 1316 is cold when added to the first formaldehyde rich gas 1330 to obtain a second cool formaldehyde reactor feed gas 1332 which is directed to the final enclosure of the pseudo-isothermal formaldehyde reactor 1322 from which the final formaldehyde rich gas 1334 is withdrawn. The formaldehyde rich gas 1334 is directed to an absorber from which the formaldehyde product and the recycle gas are obtained.

The cooling medium 1324 of the pseudo-isothermal reactor 1322 is water, which exits the pseudo-isothermal reactor 1322 in form of steam 1326 and is directed to a steam drum 1328, in which the pressure is controlled to indirectly control the temperature of the boiling water inside the pseudo-isothermal reactor.

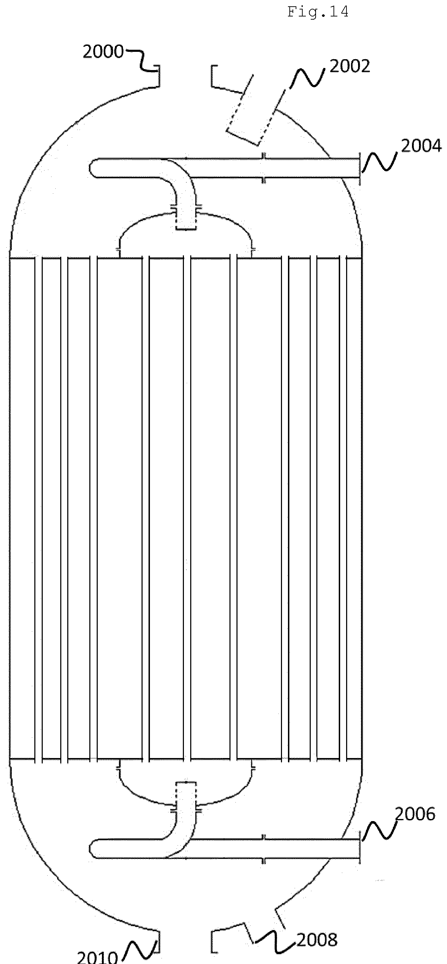

In Fig.14 a specific design of a dual-stage pseudo-isothermal reactor is shown (with cooling medium connections omitted for simplicity). Here manways 2000 and 2010 are provided for allowing access to the tubesheets and the inlet to the reaction enclosure. Gas inlets 2002 and 2004 and gas outlets 2006 and 2008 to and from the reaction enclosures are provided. In this manner access may be provided to service tube sheets and for loading and unloading of catalysts. Preferably catalyst may be unloaded by removing the inlet manifold and vacuuming from the above, with the associated benefit of avoiding the removal with inert material in the outlet manifold.

### Examples

In the following examples a number of studies of processes implemented in a reactor according to the present disclosure or reported for comparative purposes are shown. The data reported, especially the concentrations are limited only to the parameters of relevance to the illustration of the principles of the present disclosure.

### Example 1

A first series of examples relate to methanation of a biogas comprising 20.1% CH₄, 15.5% CO₂, 62.0% H₂ and 1.6% H₂O. Table 1 shows the composition of the feed and final product for this series of examples.

**Table 1**

| | **Feed** | **Ex. 1A** | **Ex. 1B** | **Ex. 1C** |
|---|---|---|---|---|
| CH₄ | 20.1 | 94.9 | 96.7 | 96.9 |
| CO₂ | 15.5 | 0.6 | 0.3 | 0.2 |
| H₂ | 62.0 | 2.3 | 0.9 | 0.7 |
| H₂O | 1.6 | 0.4 | 0.4 | 0.4 |

In a first example (Example 1A) according to the process shown in Fig. 3, methanation of a biogas takes place in four stages. As shown in Table 1 the final product has a methane concentration of 94.9%.

In a second example (Example 1B) according to the process in Fig. 4, methanation of a biogas takes place in a first pseudo-isothermal reactor stage followed by condensation and final methanation in an adiabatic reactor. As shown in Table 1 the final product has a methane concentration of 96.7%.

In a third example (Example 1C) according to the process shown in Fig. 6, methanation of a biogas takes place in a two stage pseudo-isothermal reactor according to the present disclosure. As shown in Table 1 the final product has a methane concentration of 96.9%. The performance in a process as illustrated in Fig. 5 and Fig. 6 is essentially identical, but the two separate pseudo-isothermal reactors involve higher costs.

The three examples demonstrate the improved operation when controlling the temperature of an exothermal equilibrium limited reaction such as methanation in a pseudo-isothermal reactor. Example 1B demonstrates that four adiabatic reactors can be replaced by a combination of a pseudo-isothermal reactor and an adiabatic reactor with an increase in methane concentration from 94.9% to 96.7%, in combination with a decrease in costs due to a reduction in the number of reactors. Example 1C demonstrates that a further increase to 96.9% is possible when using an integrated two stage pseudo-isothermal reactor. If Example 1C is implemented in two individual pseudo-isothermal reactors the increased complexity of a pseudo-isothermal reactor increases the costs, but if Example 1C is implemented in a single reactor the solution is estimated to be cost neutral or even less expensive.

### Example 2

In a second example shown in Table 2 according to the process illustrated in Fig.7, methanation of a synthesis gas takes place in a three stage pseudo isothermal reactor used as illustrated in Fig. 7. In the first stage, water gas shift reaction takes place, and in the second and third stages methanation takes place with intermediate condensation of water.

Example 2 demonstrates the benefit of reducing the need for preheating the gas prior to the desulfurization and methanation, and furthermore a synthetic natural gas with high methane content is obtained.

**Table 2**

| Composition [mole%] | 702 | 706 | 708 | 728 | 748 |
|---|---|---|---|---|---|
| CH₄ | 15.0 | 27.9 | 27.9 | 95.6 | 98.6 |
| CO | 20.0 | 14.0 | 5.5 | 0.0 | 0.0 |
| CO₂ | 1.0 | 0.8 | 9.4 | 0.7 | 0.1 |
| H₂ | 63.9 | 45.4 | 54.0 | 2.9 | 0.6 |
| H₂O | | 11.6 | 3.1 | 0.2 | 0.2 |

### Example 3

A third series of examples relate to methanation of a coke oven gas comprising 1.7% C₂H₆ and 1.0% C₃H₈ in the presence of steam. As shown in WO2012/084076, a process must either be operated at low temperatures or at high steam levels to operate without risking carbon formation in such a presence of higher hydrocarbons.

**Table 3**

| | **Feed** | **Ex. 3A** | **Ex. 3B** |
|---|---|---|---|
| **CH₄** | 21.8 | 72.7 | 72.8 |
| **CO** | 8.8 | | |
| **CO₂** | 4.2 | | |
| **H₂** | 57.6 | 18.6 | 18.6 |
| **H₂O** | 0.3 | 0.3 | 0.3 |
| **C₂H₆** | 1.7 | 0.0 | 0.0 |
| **C₃H₈** | 1.0 | 0.0 | 0.0 |
| **Temperature** | 300°C | 560°C | 300°C |
| **Feed flow** | | 228950 Nm³/h | 137787 Nm³/h |

In an example (Example 3A), coke oven gas is reacted to form synthetic natural gas in a process as illustrated in Fig. 8, where the process is operated in three adiabatic reactors in series with a maximum temperature of 560°C, which may leave no margin or a very small margin to the carbon formation limit. The resulting volumetric flow of gas into the first methanation reactor is 228950 Nm³/h. In a further example (Example 3B), coke oven gas is reacted to form synthetic natural gas in a process as illustrated in Fig. 9, where the process is operated in a dual stage pseudo-isothermal reactor with a maximum temperature of 300°C, which leaves a wide safety margin to the carbon formation limit. The resulting volumetric flow of gas into the first methanation reactor is 137787 Nm³/h, which is only 60% of the flow required in Example 3A.

A comparison of Example 3A and Example 3B shows that the operation in a dual stage pseudo-isothermal reactor provides ample safety in operating conditions to the carbon formation limit of a typical methanation catalyst, whereas operation in a three stage adiabatic configuration results in a significant risk of carbon formation.

### Example 4

A fourth series of examples relate to the production of 4 t/h of methanol from a synthesis gas comprising 29.9% CO and 66.5% H₂. The corresponding data are shown in Table 4.

**Table 4**

| | **Feed** | **Ex.4A** | **Ex.4B** | **Ex.4C** |
|---|---|---|---|---|
| **CH₃OH** | | 98.4 | 97.7 | 96.8 |
| **CH₄** | 1.1 | 0.0 | 0.0 | 0.0 |
| **CO** | 29.9 | 0.0 | 0.0 | 0.0 |
| **CO₂** | 1.7 | 0.3 | 0.2 | 0.2 |
| **H₂** | 66.5 | 0.1 | 0.1 | 0.1 |
| **H₂O** | 0.1 | 1.3 | 2.0 | 2.9 |
| **Flow** | | 9634 Nm³/h | 9262 Nm³/h | 9192 Nm³/h |

In a first methanol production example (Example 4A, cf. Fig. 10) a configuration of a single pseudo-isothermal methanol reactor with recycle according to the prior art is used for methanol production. According to this example 9,634 Nm³/h of synthesis gas are required for the production of 4 t/h methanol.

In a second methanol production example (Example 4B, cf. Fig. 11) a three stage pseudo-isothermal reactor according to the present disclosure is used for methanol production. According to this example 9,262 Nm³/h of synthesis gas are required for the production of 4 t/h methanol.

In a third methanol production example (Example 4C, cf. Fig. 12) a two stage pseudo-isothermal reactor with recycle according to the present disclosure is configured with recycle used for methanol production. According to this example 9,192 Nm³/h of synthesis gas are required for the production of 4 t/h methanol.

The comparison of these three examples demonstrates that methanol production in a pseudo-isothermal reactor is very efficient, and also that this may beneficially be implemented in a reactor according to the present disclosure.

### Example 5

In Table 5 the composition and temperature of the flows of a formaldehyde production corresponding to Fig. 13 are shown.

By using a dual stage pseudo-isothermal reactor it becomes possible to add methanol in two steps and to quench the product of the first stage by addition of cold methanol.

If the formaldehyde production process was carried in a single stage process the concentration of methanol would be kept low by recycle of product. However, this recycle requires additional equipment volume, as well as energy for recycle compressors, and therefore it would be associated with an extra cost.

**Table 5**

| Composition [mole%] | 1^{st} reactor feed | 1^{st} reactor product | 2^{nd} reactor feed | 2^{nd} reactor product |
|---|---|---|---|---|
| O₂ | 14.5 | 11.1 | 10.2 | 5.8 |
| CO | 0.5 | 0.8 | 0.7 | 1.1 |
| CH₃OH | 6.0 | 0.1 | 8.0 | 0.1 |
| CH₂O | | 5.4 | 5.0 | 11.9 |
| H₂O | 4.2 | 10.0 | 9.3 | 16.8 |
| Temperature | 207°C | 285°C | 165°C | 285°C |

## Claims

1. A process for production of methanol in a multiple reaction enclosure pseudo-isothermal reactor, said process comprising the steps of
a) directing a synthesis gas to contact a first material catalytically active in methanol synthesis inside a first reaction enclosure,
b) withdrawing a first gas rich in methanol from said first reaction enclosure,
c) cooling said first gas rich in methanol and directing it to a gas/liquid separator,
d) withdrawing a condensate and an intermediate process gas from said separator,
e) directing said intermediate process gas to contact a second material catalytically active in methanol synthesis inside a second reaction enclosure, and
f) withdrawing a second product gas rich in methanol from said second reaction enclosure.

2. A process for production of methanol according to claim 1, said pseudo-isothermal reactor further comprising a sulfur guard reaction enclosure comprising a material active in desulfurization for withdrawing sulfur from said synthesis gas upstream said other reaction enclosures.

3. A process plant for production of methanol according to claim 1 or 2 comprising a pseudo-isothermal flow reactor for an exothermal reaction comprising at least two reaction enclosures and a cooling medium enclosure configured to hold a cooling medium under pressure at the boiling point of said cooling medium, said reaction enclosures having an outer surface configured to be in thermal contact with the cooling medium, each of said reaction enclosures having a reaction enclosure inlet and a reaction enclosure outlet and said cooling medium enclosure having a cooling medium inlet and a cooling medium outlet and each of said inlets and outlets being individually connectable.

4. A process plant for production of methanol comprising a reactor according to claim 3 further comprising a catalytically active material inside at least 50% or 80% of the volume of at least one reaction enclosure.

5. A process plant for production of methanol comprising a reactor according to claim 3 or 4, further comprising an inlet manifold wherein at least one of said first reaction enclosure and said second reaction enclosure comprises a multitude of reaction tubes, such as at least 2, 50, 100 or 1000 reaction tubes, each tube having a tube inlet in fluid connection with said inlet manifold, which is configured to receive a fluid stream from said reaction enclosure inlet and to distribute said fluid stream between the tube inlets of said multitude of reaction tubes.

6. A process plant for production of methanol comprising a reactor according to claim 3, 4 or 5, further comprising an outlet manifold wherein at least one of said first reaction enclosure and said second reaction enclosure comprises a multitude of reaction tubes, such as at least 2, 50, 100 or 1000 reaction tubes, each tube having a tube outlet in fluid connection with said manifold, which is configured to receive a fluid stream from each of said multitude of tube outlets, said manifold combining the multitude of fluid streams into a single fluid stream and directing the single fluid stream to said reaction enclosure outlet.

7. A process plant for production of methanol comprising a reactor according to claim 5 or 6 comprising a reactor divider separating the cooling medium from a reaction space in fluid connection with a reaction enclosure inlet or a reaction enclosure outlet and at least two reaction tubes of said reaction enclosure, thus defining an inlet manifold or an outlet manifold of said reaction enclosure.

8. A process plant for production of methanol comprising a reactor according to claim 5 or 6 in which said manifold comprises at least two substantially cylindrical manifold chambers, each manifold chamber being in fluid connection with the reaction tubes of a single reaction enclosure, and said two manifold chambers not being in direct fluid connection with each other, wherein said two cylindrical manifold chambers are positioned substantially concentrically.

9. A process plant for production of methanol comprising a reactor according to claim 5 or 6 in which said manifold comprises at least two manifold chambers, each manifold chamber having the shape of an angular cylinder sector, and each manifold chamber being in fluid connection with the reaction tubes of a single reaction enclosure while not being in direct fluid connection with each other, and in which said at least two manifold chambers together define a cylindrical shape.

10. A process plant for production of methanol comprising a reactor according to claim 5, 6, 7, 8 or 9, further comprising a means of fluid connection, said means of fluid connection being connected to a boundary wall of said reactor shell and to the inlet manifold or the outlet manifold, said means of fluid connection being configured to allow thermal contraction and expansion by a design such as a U tube, or an angular tube.

11. A process plant for production of methanol comprising a reactor according to claim 3, 4, 5, 6, 7, 8, 9 or 10 further comprising a vapor drum having a vapor drum inlet and a vapor drum outlet, said a vapor drum inlet being in fluid communication with said cooling medium outlet by a riser tube and said vapor drum outlet being in fluid communication with said cooling medium inlet by a downcomer tube and said vapor drum being positioned above said cooling medium enclosure.

12. A reactor section comprising a reactor according to claim3, 4, 5, 6, 7, 8,9 ,10 or 11, said reactor having a first reaction enclosure and a second reaction enclosure, and said reactor section further comprising a cooler and a gas/liquid separator having an inlet, a gas outlet and a liquid outlet, said reactor section being configured such that
a) the cooler inlet is in fluid communication with the first reaction enclosure outlet,
b) the cooler outlet is in fluid communication with the separator inlet, and
c) the separator gas outlet is configured to be in fluid communication with the second reaction enclosure inlet.
